# EUROPEAN PATENT SPECIFICATION

(11) **EP 2 007 448 B1**
(45) Date of publication and mention of the grant of the patent: **22.01.2014**
(21) Application number: 07748391.5
(22) Date of filing: 11.04.2007
(51) Int. Cl.: A61L 29/14, A61M 25/02, A61M 25/06, A61L 29/06, A61L 29/12

(54) **CATHETER TUBING, CATHETER, AND CATHETER ASSEMBLY**
KATHETERRÖHRE, KATHETER UND KATHETERANORDNUNG
TUBE DE CATHÉTER, CATHÉTER ET ENSEMBLE CATHÉTER

(30) Priority: 19.04.2006 SE 0600874
(43) Date of publication of application: 31.12.2008
(73) Proprietor: Nordic Med -com Aktiebolag, 503 13 Borås (SE)
(72) Inventor: PUHASMÄGI, Arne, 503 33 Borås (SE); SVEDBERG, Michael, 302 41 Halmstad (SE); GRINNEBACK, Kay, 442 31 Kungälv (SE)
(74) Representative: Johansson, Lars-Erik
(86) International application number: PCT/SE2007/050230
(87) International publication number: WO 2007/120109

(56) References cited:
- EP-A2- 0 341 049
- EP-A2- 0 472 413
- US-A- 4 846 812
- US-A- 5 266 669
- US-A- 5 453 099
- LI S ET AL: "Cross-linking kinetics and swelling behaviour of aliphatic polyurethane", POLYMER, ELSEVIER SCIENCE PUBLISHERS B.V, GB, vol. 41, no. 15, 1 July 2000 (2000-07-01), pages 5571-5576, XP004193866, ISSN: 0032-3861, DOI: 10.1016/S0032-3861(99)00785-5

## Description

### TECHNICAL FIELD

The invention relates to a catheter tubing, which means a tubing intended to be used for the manufacturing of catheters, and a catheter made of such catheter tubing, particularly for hemo-dialysis.

The disclosure also relates to a catheter assembly, which may comprise a catheter of that type, particularly a fistula catheter, but which alternatively may comprise other catheters than fistula catheters for hemo-dialysis. The assembly in both cases comprises a proximate conduit and a hub forming a connection between the catheter and the proximate conduit for conveying blood or other liquid to or from a patient, said hub comprising a rear portion through which there extends a straight passageway, which is coaxial with an end portion of the catheter, which is connected to the hub, and a branch conduit to which said proximate conduit is or can be connected, and a distal portion in front of the mouth of said branch conduit. Particularly, the invention concerns a fistula catheter and a catheter assembly, respectively, intended to be used in connection with hemo-dialysis.

### BACKGROUND OF THE INVENTION

Kidney malfunction often requires blood dialysis, usually hemo-dialysis. This treatment must be made 3-5 times a week and their time duration is 3-5 hours at every occasion. Only in Sweden, about 350,000 such dialysis are made every year. To be able to connect the dialysis equipment to the blood circulation system of the patient, a blood vessel is modified by connecting an artery to a vane. After maturation the veinis expanded to a fistula, which is suitable for inserting the two catheters needed. The frequent penetration of the fistula and the damage caused by the mechanical contact by the catheters make the fistula age so that it gets ripple. The aged fistula does not heel but must be replaced by a new one after a couple of years. The human body has only eight positions considered suitable for making fistulas. When they are all aged, more dangerous dialysis methods must be used as a last care.

Plastic catheters are generally known and have in comparison with catheters made of steel the advantage that they are softer, but they do not satisfy ever increasing demands as far as care of the skin of the patient are concerned when used as fistula catheters. The US patent 7 094 243 discloses a catheter which has an expandable end portion, which can deposit or take samples etc., and WO 2004/037333 discloses a catheter having a very high elasticity, which can be used as a conveyor for inserting major objects into the human body through the fact that the catheter can be widened by said objects as they are pressed through the catheter. None of these catheters can be employed in connection with dialysis treatment and they do not solve any of those problems which the present invention aims at solving.

US5453099 discloses a catheter tubing that has a layer of a hydrophobic stiffening polymer encapsulated by a layer of hydrophilic thermoplastic base material. The encapsulated layer may be stripe or an annular layer having base polymer layers laminated on both surfaces thereof.

Further, a catheter assembly of the type mentioned in the assembly is known through the US patent 4 955 863. This assembly comprises a pair of wings which can be used as a gripping member during the operation of the assembly and also for the fixation of the catheter against the skin. Also the US patent 3 589 361 shows a similar gripping and securing member. It is a typical feature of these members, however, that they are not mounted on or form an integrated part of the hub that constitutes a separate unit which is mounted on the catheter itself, which does not promote a care handling of the assembly during the penetration of the patient's skin or during the continued treatment.

### DESCRIPTION OF THE INVENTION

Even if a catheter designed according to the invention can be employed also for other purposes than as a fistula catheter, the invention in the first place aims at providing an improved fistula catheter and a catheter tubing intended for such type of a catheter. In connection herewith it is a purpose of the invention to make a delay of the aging of the fistulas possible. This can be achieved according to the invention along, namely;
the development of a catheter tubing and of a catheter made of such catheter tubing, which is more kind to the patient's skin in the region of said fistulas and therefore more fitted to be employed as a fistula catheter, and/or provides an opportunity to make the total time duration for each dialyse shorter.

The catheter tubing and the catheter made of such tubing consist of a composite, comprising a first material which consists substantially of a hydrophilic plastic material which absorbs moist and therefore swells such that its volume will increase significantly in contact with e.g. blood, and a second material which essentially does not absorb moist and therefore will not swell in a moist environment, but which forms reinforcing, longitudinal strands in the catheter tubing, which counteract that the catheter tubing is elongated or that its thickness is increased due to the swelling capacity of the first polymeric material, such that the increase of the volume of the hydrophilic first plastic material substantially results in an increase of the circumference of the tubing and hence of its diameter and duct cross section area. Through the widening of the catheter, opportunities are afforded to use a catheter which initially is more narrow, which per se reduces the risk for damagement of the fistula, at the same time as the widening allows the same blood flow to be maintained as in an initially somewhat larger catheter, or allows the use of a catheter having an initially normal dimension which is widened during the dialysis operation so that the blood flow is increased and thence the dialysis duration is made shorter, in those cases this is possible from a medical point of view. Compromises between these choices are also possible which create opportunities for more optimal dialysis conditions than have been possible in the past and/or create possibilities to reduce the number of standardised catheter dimensions, which is advantageous from an economical point of view.

Typically also said first, swellable material has the shape of longitudinal strands. In principal one may conceive that the swellable plastic material extends all around the catheter tubing, in other words that it has the character of a single strand in which longitudinal strands or fibres of the reinforcing non-swellable material are embedded, which essentially prevents the tubing from expanding in the axial direction in an aqueous environment, i.e. that it will be extended.

Preferably, however, the strands of the first and of the second material in cross-section form segments of an annular ring, which sections are connected edge-edge to one another, i.e. such that each strand of the first material on each side thereof has a strand of the second material. This not only counteract an elongation of the tubing because of the reinforcing strands of said second material but also that the strands of said second material prevents a swelling in the radial direction of the swellable material in the strands of the first material. This result, however, is restricted to the narrow zones of the swellable material which border on the segments of the none-swelling material. In order that the effect shall be desirably large, the segments of the swellable material therefore ought to be so thin that the counteracting influence of the non-swelling elements upon the radial swelling of the swellable segments shall have an impact upon the entire swellable segments, in other words such that substantially the entire width of the swellable segments will constitute zones within which swelling in the radial direction is essentially prevented. In this case, the reinforcing, non-swelling strands can counteract any increase of the swellable strands in the radial direction, so that the volume increase of the swellable strands occur substantially in the circumferential direction, i.e. mainly in only one, desired direction. But if the swellable segments need to be thin in order to provide the desired effect they also need to be correspondingly many in order that the total circumferential length of the swellable segments shall be sufficient in order to provide the total swelling in the circumferential direction that is desired. The number of segments of said first and second materials therefore should be at least 10, preferably at least 15, and most advantageously at least 20 of each kind. Preferably, at least the swellable strands which in cross-section have the shape of segments of an annular ring initially have a width, which means extension in the circumferential direction, in the same order as the wall thickness of the catheter, preferably a width which is smaller than the wall thickness, such that they have the character of lamellas.

Other important factors for the design of a catheter tubing having an optimal combination of features are; the swelling capacity of the swellable material, the portions of swellable and non-swellable materials in the tubing, the stiffness and softening of the catheter subsequent to its penetration into the human body, and the duration of the dialyse in the case of a fistula catheter with reference to possible, time-dependent changes of the catheter.

As far as the swelling capacity of the hydrophilic plastic material which is included in said first material is concerned, it should first be mentioned that it is true that many, maybe even most plastic materials, have some moist absorbing capacity and therefore also some swelling capacity in an aqueous environment, but it is also true that this swellability is not significant, but is to be regarded as negligible for most areas of employment or at least comparatively low for plastic materials which are not classified as hydrophilic, moist absorbing and/or swelling in an aqueous environment. This is well know to the man skilled in the art and if more specific characteristics are needed for the implementation of the invention in accordance with what is taught by this patent description, by combining swellable and non-swellable plastic material in order to achieve a combination of swellability in the circumferential direction and consequently also in the radial direction and an adequate stiffness, which is desired for catheter tubings, any desired product information may be obtained from the manufacture of the material in question, which the man skilled in the art also knows. What is meant with non-swellable polymeric plastic materials because of insignificant absorption of water should not need to be defined more precisely. However, it is stated in this text that with the "materials which do not essentially absorb water and therefore do not swell in an aqueous environment" shall mean materials which have a maximal swellability of 2 %, preferably not more than 1 %, most advantageously not more than 0.5 in any direction after a period of duration of 1 hour in water having a temperature of 36 °C.

There exist plastic materials having a swellability all the way up to 800 %, i.e. having the capacity to increase its volume up to eight times through the absorption of water, as for example some polyurethane based, hydrophilic materials. In principal it is possible to employ a plastic material for said first material having such high swellability, if the segments of the first materials are made much thinner than the segments of the second, reinforcing material. However, a material having such a high water absorption will soften in a very high degree. To this shall be added the softening which depends on the higher temperature in contact with liquids in the human body. The swellability of the hydrophilic, swellable plastic material which is included in said first material therefore should not exceed 200 %, preferably not exceed 100 %. In order to provide the desired effect, the swellability of the swellable plastic material should be at least 10 %, preferably at least 15 %. A suitable swellability may lie in the range 15-60 %.

Polyurethanes and polyamides are examples of plastic materials which can be afforded hydrophilic features. Among polyurethanes which because of absorption of water swell very much in aqueous environments may be mentioned hydrophilic and swellable grades manufactured by Noveon Inc., Cleveland, Ohio, US with the trade name Techophilic^{®}. In Table 1, below, a number of conceivable plastic materials are listed, which should be possible to be employed as a swelling component in the catheter tubing according to the invention. The trade names of the materials are given in the table, their stiffness at 20 °C and at 36 °C, and the stiffness ratio 20 °C/36 °C. The importance of the stiffness will be discussed in the following in connection with the disclosure of conceivable, reinforcing, non-swelling plastic materials in connection with Table 2.

**Table 1**

| Swellable plastic materials | | | |
|---|---|---|---|
| Techophilic grade | Stiffness at 20 °C | Stiffness at 36 °C | Stiffness ratio at 20 °C/36 °C |
| 100 | 9.14 x 106 | 7.01 x 10⁶ | 1.30 |
| TG 500 | 7.53 x 10⁷ | 2.64 x 10⁷ | 2.85 |
| HP 60D-60 | 7.81 x 10⁷ | 2.27 x 10⁷ | 3.44 |

Through the choice of grade of swellable material and through the choice of the total circular arch length of those segments which consist of said first material, which comprises the swellable plastic material, in relation to the total arch length of the non-swellable segments, the expansion capacity of the catheter can be varied to a high degree. For example, the total portion of the circumference of the swellable strands of the catheter initially may represent 2-98 % of the circumference, or preferably 5-50 % of the circumference, suitably at least 15 %. In a preferred embodiment, the swellable strands may represent 15-40 % of the total initial circumference. The man skilled in the art therefore can tailor expandable catheters from case to case through the choice of the total portion of the cross section (the circumference) of the swellable segments and/or their swellability.

The longitudinal, reinforcing strands of said second material, which does not absorb water, has one more mission, besides directing the increase of volume of the swellable strands such that it will result in an increase of the volume mainly in the circumferential direction of the tubing, namely to afford the catheter an adequate stiffness. Adequate stiffness in this connection shall mean such a high initial stiffness of the catheter that it can penetrate the skin of the patient without folding at the rear of the cannula, in combination with a sufficient stiffness of the catheter also after a duration for a period of time in the warm, aqueous environment in a fistula or other vessel of a patient in order that the catheter shall not easily be clamped together or kink. "Sufficient stiffness" in this connection does not exclude that the catheter will soften to a certain degree. To the contrary, this is desired in order that the catheter shall be as kind as possible against the patient's skin. In this connection, the swellable, hydrophilic plastic material in the swellable strands in the first place will be responsible for the desired softening, while the reinforcing strands of said second material will moderate this softening by remaining comparatively stiff in spite of the higher temperature. The latter feature is expressed in terms of stiffness ratio at 20 °C/36 °C. According to this aspect of the catheter tubing and the catheter according to the invention, the stiffness of the reinforcing second material expressed as Pa at 20 °C should be at least 10⁸ Pa, preferably at least 5 x 10⁸ Pa, most conveniently at least 10 x 10⁸ Pa, and the stiffness ratio 20 °C/36 °C, i.e. the ratio between the stiffnesses at 20 °C and 36 °C, should be at most 2, preferably at most 1.5. In Table 2, below, two commercial aliphatic, polycarbonate based plastic materials of the polyurethane type are given, which may be conceived to be employed as main component in said second material. The Carbothane^{®} material is manufactured by Dow Chemical Company, Midland, Michigan, USA, and the Pellethane^{®} material is manufactured by the previously mentioned Noveon Inc.

**Table 2**

| Stiffening plastic materials | | | |
|---|---|---|---|
| | Stiffness at 20 °C | Stiffness at 36 °C | Stiffness ratio at 20 °C/36 °C |
| Carbothane 72C | 8.55 x 10⁸ | 4.60 x 10⁸ | 1.86 |
| Pellethane 2363-75D | 14.90 x 10⁸ | 55.59 x 10⁸ | 1.29 |

It has been mentioned in the foregoing that the reinforcing strands of said second material has several important functions; to make the catheter stiffer, to counteract axial expansion thereof, and to counteract that the strands of swellable material will increase in the wall thickness direction of the tubing. The two first mentioned functions, however, may at least partly be taken over by reinforcing fibres and/or strands of stronger material which are imbedded in the swellable plastic material, and possibly also, or as an alternative, in the non-swelling strands. Among conceivable, reinforcing materials may be mentioned e.g. vectran fibres, aramide fibres (Kevlar), high module fibres, such as carbon fibres and glass fibres, and others.

The catheter tubing, which consists of a composite may, whether the composite contains the just mentioned reinforcing materials or not, be manufactured through established techniques, such as co-extrusion or pultrusion of included components.

The catheter, according to the preferred embodiment, extends through an opening in the front end of the hub and at least a distance into the hub, in which it is secured. Preferably the catheter conduit, i.e. the passageway defined by the catheter, changes inside of said opening into a passageway in the main body of the hub, said passageway extending all the way to said proximate conduit, wherein the cross section of the passageway for conveying blood or dialyse liquid, defined by a portion of the catheter in the hub and by said previously mentioned passageway, will increase without any sharp changes of the cross section in the region between the opening and the proximate conduit. This provides an even conduit without abrupt changes of the cross section and thence an even flow path for the blood which during the dialyse shall be conveyed through the catheter assembly. This contributes to a careful conveying of the blood liquid which reduces the risk for haemolysis because of turbulence in the liquid.

Advantageously, the above mentioned avenues of development can be combined in the catheter assembly according to the invention. Thus, the catheter in the catheter assembly according to a preferred embodiment of the invention at least partly consists of a hydrophilic plastic material which swells significantly in contact with liquids of the human body, including blood. Preferably, the catheter consists of a catheter according to the invention. Therefore the outer diameter of the catheter, according to this preferred embodiment, initially is smaller than the diameter of the opening in the front end of the hub, such that the outer diameter of the catheter can expand through swelling in use without obstacle caused by the hub in the region of the opening. Suitably, the diameter of the opening is so much larger than the catheter that the catheter will completely fill the opening due to swelling without constriction by the hub material in the region of the opening.

Further characteristics and aspects of the invention will be apparent from the following, more detailed description and by the appending patent claims.

### BRIEF DESCRIPTION OF DRAWINGS

In the following detailed description, reference will be made to the accompanying drawings, in which
Fig. 1 is a view from above, showing a catheter assembly according to an embodiment of the invention, prior to use of the assembly,
Fig. 2 shows the assembly in a view from below,
Fig. 3 shows the assembly from behind in a view along the line III-III in Fig. l,
Fig. 4 shows the assembly in cross section along the line IV-IV in Fig. 3,
Fig. 4A and Fig. 4B show parts of the assembly in cross section at a larger scale before and after, respectively, a cannula, which initially is included in the assembly, has been removed,
Fig. 4C shows an encircled detail in Fig. 4B at a still larger scale,
Fig. 5 shows the assembly during treatment of a patient after the cannula has been removed,
Fig. 6 is a perspective view of an element included in the assembly,
Fig. 7 shows a longitudinal cross section through a front section of a catheter assembly according to a second, more preferred embodiment of the invention,

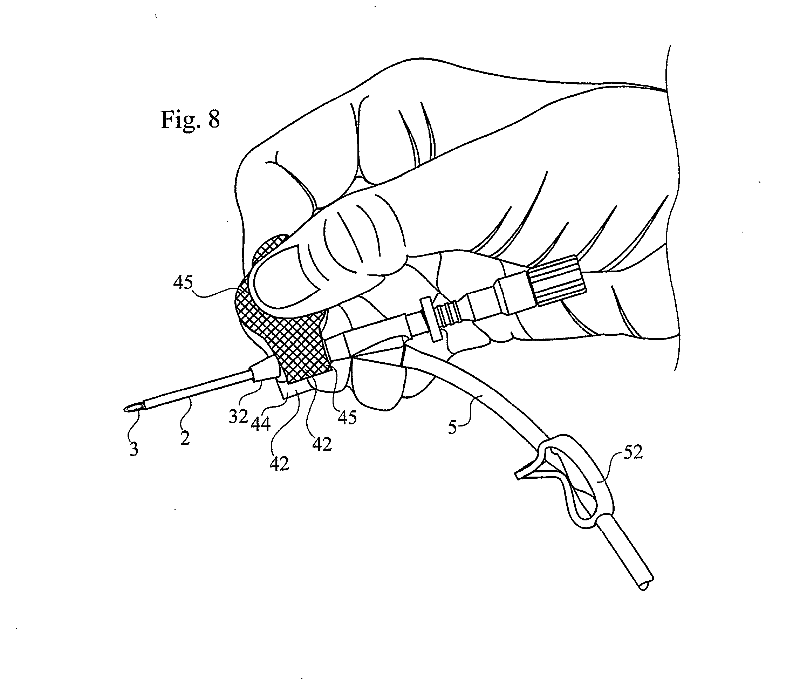

Fig. 8 illustrates how an assembly according to the invention is operated when the catheter shall be inserted into a fistula,
Fig. 9 shows an enlarged perspective view of a catheter according to a preferred embodiment of the invention,
Fig. 10A and 10B show, at a still larger scale, a portion of the catheter according to Fig. 9 in cross section before and after, respectively, swelling of the water absorbing material,
Fig. 11 illustrates a developed embodiment of the catheter according to Fig. 9 and 10,
Fig. 12 illustrates an experiment, in which a pile of sheets of swelling and non swelling plastic materials were laminated to form an integrated pack, and
Fig. 13 shows a slice made of the pile according to Fig. 12 through cutting or sawing, intended to be used as an object of the experiment.

### DESCRIPTION OF PREFERRED EMBODIMENTS

### CATHETER TUBING AND CATHETER

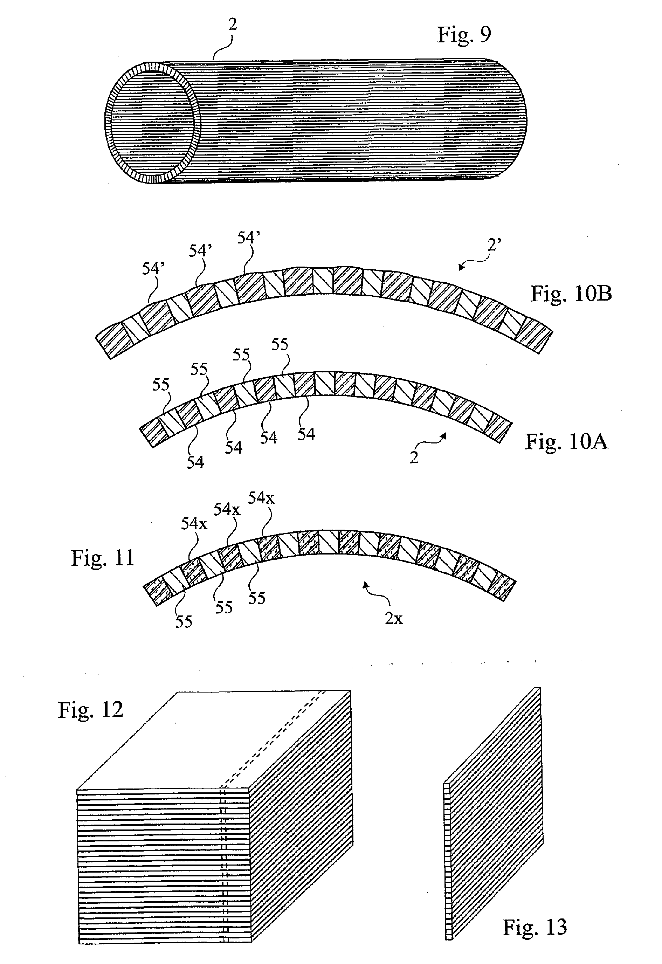

Fig. 9 shows a conceivable embodiment of a catheter 2 according to the invention, before it has been inserted into a fistula or other blood vessel and been brought into contact with blood at the temperature of the human body. Fig. 10A shows a portion of the catheter in cross section at a larger scale. The catheter consists of strands 54 of a hydrophilic, water absorbing plastic material, such as any of the polyurethane based plastic grades which are known under the trade name Techophilic^{®}, which are listed in Table 1, alternating with strands 55 of a stable, non-water absorbing plastic material, preferably of the same polymer family as the hydrophilic strands 54, i.e. a polyurethane in the example in question, e.g. any of those plastic materials which are listed in table 2. By choosing closely related plastic grades, a safe edge uniting of the strands, which in cross section have the shape of segments of a circular ring, is facilitated, as is shown in Fig. 9. Established technology is available for the manufacturing of composite plastic tubings of very small dimensions. E.g. polyurethanes may be co-extruded to form composite tubings suitable to be employed as catheters, containing embedded strands of barium sulphate for x-ray detection. Such technology may be applied for the manufacturing of the catheter 2 according to the invention, which means that the strands 54 and 55 are united to one another in a molten condition of the materials.

In the example which is illustrated in Fig. 9, the number of strands 54 and 55 is very large; forty strands of each kind, which initially have equal width in the circumferential direction. As is shown in Fig. 10A the swellable 54 as well as the non-swellable strands 55 initially have the shape of lamellas which is typical for the invention.

In Fig. 10B also the pronounced increase of the diameter of the catheter 2 is illustrated. Such increase of the diameter is achieved by even a moderate increase of the volume, about 30 %, of the segments/strands 54, which in the swollen catheter 2' are designated 54'. The stiffening segments 55, which do not consist of water absorbing material, on the other hand remain intact and counteract an elongation of the segments 54 and thence also of the entire catheter 2. They also counteract that the segments 54 will be thicker, i.e. that the extension in the radial direction of the individual segments 54 will increase. The restraining effect of the stiffening segments 55 upon the swelling segments, however, is restricted to a zone close to the surface of contact between swelling and non swelling segments. It is understood that the construction according to the invention, which includes thin, more or less lamella shaped segments is essential in order that said restraining effect shall be achieved. In summary the reinforcing, non swelling segments 55 thus control the increase of volume of the swellable segments 54, such that it takes place substantially in the circumferential direction, as is illustrated in Fig. 10B.

Fig. 11 shows a portion of a catheter 2x in cross section, where the swellable strands/segments 54x contain reinforcing, thinner, longitudinal strands of any of the reinforcing materials which have been mentioned in the foregoing, embedded in the hydrophilic plastic material. In this way, particularly the resistance against axial expansion of the catheter because of the increase of volume of the swelling plastic material is enhanced. As has been mentioned in the foregoing it is possible, as an alternative, or in addition, that also the swellable strands 55 may be reinforced in the this mode.

### Experiments

Films were moulded by hot pressing between teflon slices to a thickness of 0.2 mm from a plastic material having a good swellability in aqueous environment and also from a non swellable plastic material. The swellable material was a polyurethane (PUR) known under its trade name Techophilic^{®} HP-93A-100, which has a solid state hardness of Shore A 93 and 100 % water absorption capacity. The stiffening, non swellable material also was a polyurethane; Carbothane^{®} PC 35-55, with a solid state hardness of 55 shore D. Fifteen slices of each material were alternatively piled one upon the other, every second of the first material and every second of the other material. The pile was dried in vacuum and was then vacuum laminated for 1h at 170 °C. The laminate which was manufactured in this mode is illustrated in Fig. 12; idealized and not quite according to scale. Slices of various thickness were made from this laminate, such that the laminate slices were made to consist of elongated, narrow strands of alternatively swellable and non swellable material, lying side by side, Fig. 13.

In a first experiment, a slice with a thickness of 1.6 mm was tested in distilled water at 23 °C. The test showed that the increase of width because of swelling was rather slow because of the comparatively great thickness of the experimental material. After 1h the increase of the width was 7.4 % which had increased to 14 % after 24h. This is too slow in consideration of the conceived field of use, haemodialysis.

In a next experiment, therefore a thinner slice having a thickness of 0.45 mm was tested, which is a thickness which is more normal for dialysis catheters, cut from the same laminate as in the previous experiment. Also in this case, the experimental medium was distilled water, 23 °C. Already after 5 min, the increase of width amounted to 15.43 %. After a period of time, also the length increased to a non-negligible degree because of a limited capacity of the chosen plastic material in the non swelling strands to withstand the stress from the swelling strands in the longitudinal direction of the sample.

A catheter tubing can be cut in the longitudinal direction to form a sheet having a thickness and width in the same order as the experimental slice in the latter experiment. The experiment therefore simulates testing of a catheter tubing. The achieved results show that the composite plastic material, which was tested in the experiment, can provide an essential increase of the diameter if it is employed for fistula catheters, and indicate that the composition can be optimized through measures taken in order to efficiently counteract the elongation of the catheter, e.g. by using a stronger material in the non swelling strands, for example the very strong polyurethane Pellethane^{®} 2363-75D, Table 2, and/or by reinforcing the swellable strands as has been mentioned in the foregoing.

### THE CATHETER ASSEMBLY

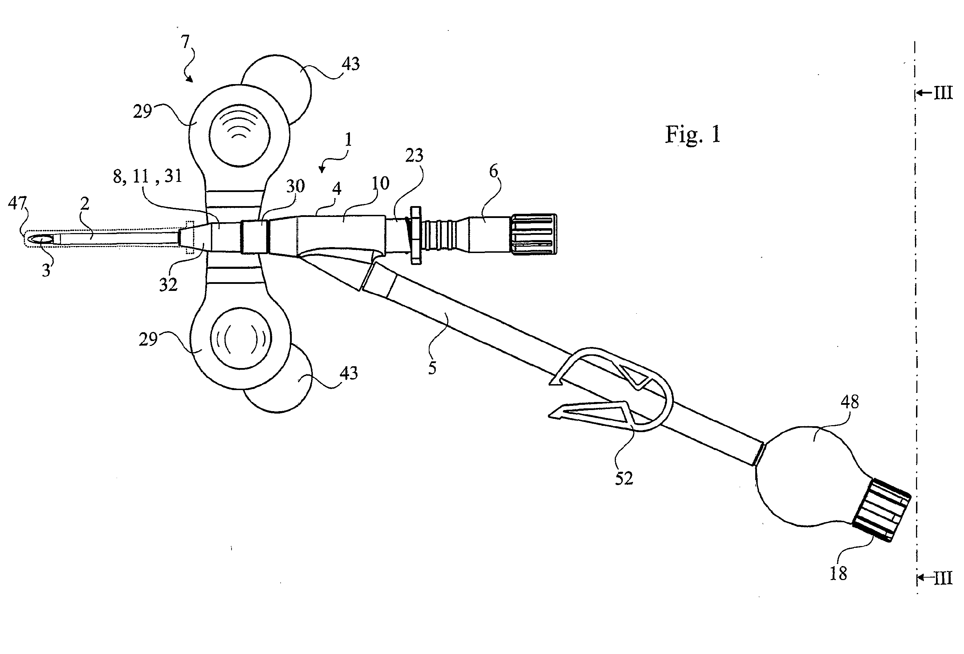

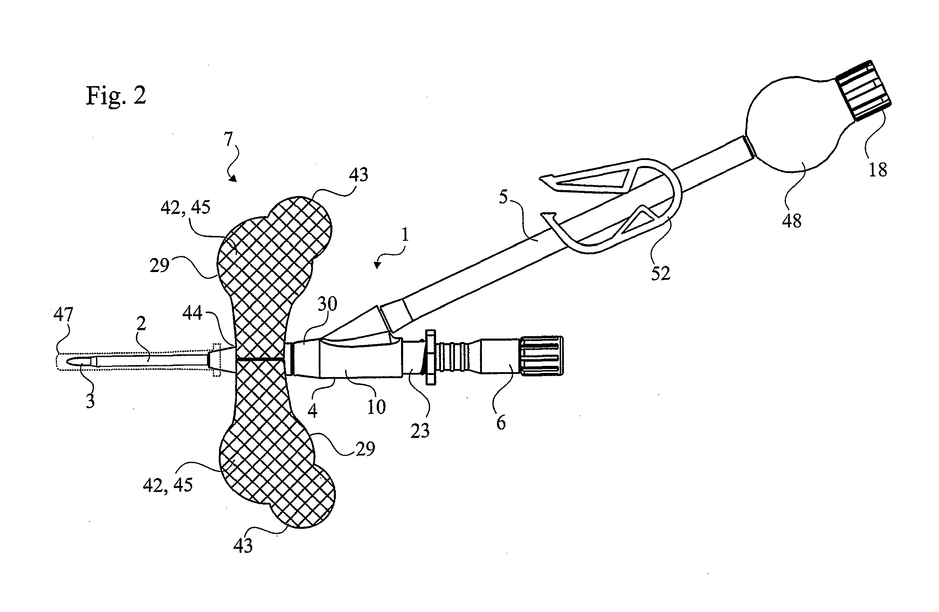

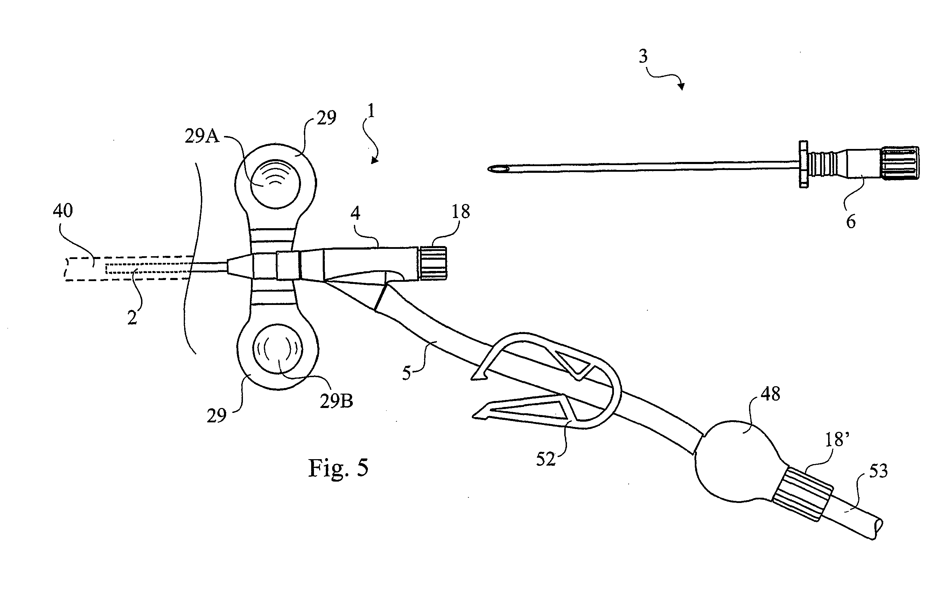

In Fig. 1-6 a catheter assembly is generally designated 1. Its main parts consist of a catheter 2, which according to the embodiment consists of a thin plastic tubing, a cannula 3 in the form of a tubular needle made of metal, which extends through the catheter 2, a connection piece, here referred to as hub 4, and a proximate conduit 5. In the rear end of the cannula 3 there is provided a back member 6. The cannula 3 with its back member 6, according to the embodiment, is of a type known per se. A gripping and fixation member is designated 7. In its package, the catheter and the cannula is covered by a protective cap 47.

The hub 4, which is made of a stiff and transparent plastic material, consists of a rear portion 10, which forms the main portion of the hub, and a distal portion 11. A first, straight, narrow passageway 12, herein referred to as cannula passageway, extends from the rear end of the hub 4, and through the rear portion 10 of the hub and mouths in the front part of a branch conduit 13. In the distal potion 11 of the hub, there is an inlet chamber 14, which forms an extension of the cannula passageway 12 and is coaxial with said passageway. The branch conduit 13 starts with a curve 15 and thereafter exhibits a straight portion 16 which extends to the proximate conduit 5. The straight portion 16 of the conduit extends obliquely rearwards relative to the straight cannula passageway 12.

In the rear hub portion 10 one or two membranes 17, each about 3 mm thick, also referred to as septum, are provided in a manner per se and made of a material which can be conventional, e.g. silicon rubber or polyisoprene and penetrated by the cannula 3 but which, when the cannula is removed, automatically seal the aperture which the cannula has created at the penetration. In the rear end 23 of the rear portion 10 there is provided, in a manner per se, an outer standard type threading for a luer-lok™ closure 18. The back member 6, includes a small chamber 19 in a manner per se in front of a membrane 20 which is permeable to air. The chamber 19 forms a terminal for the lumen 21 of the cannula 3. The membranes or the filter 20 is provided in the front end of a filter adaptor 22 in a manner known per se.

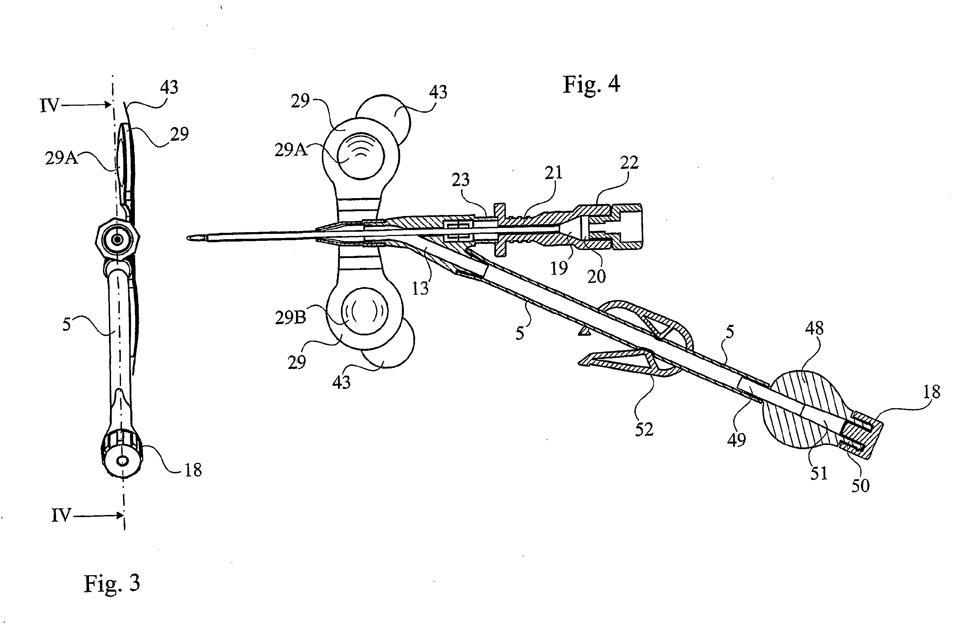

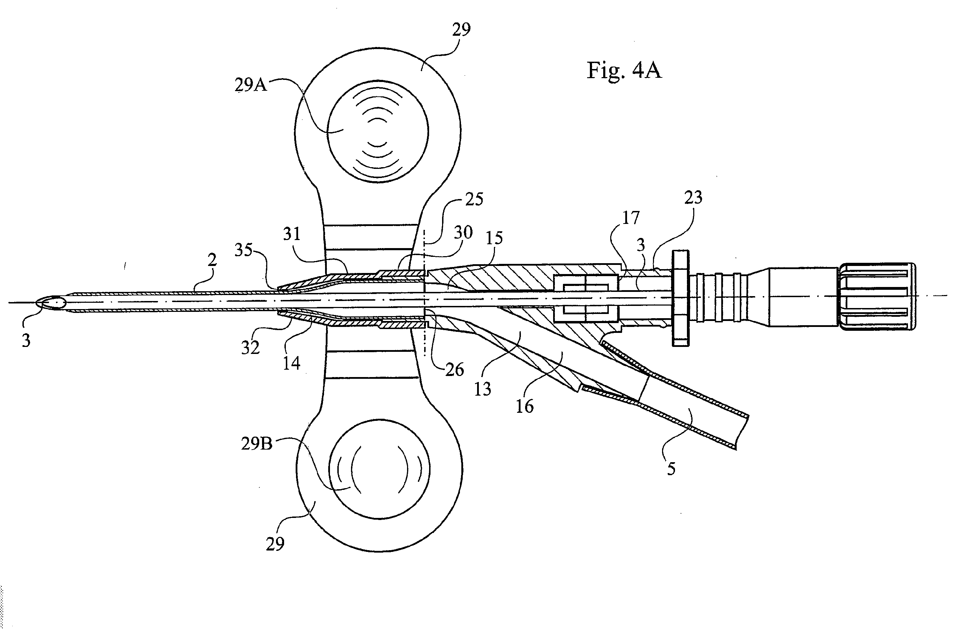

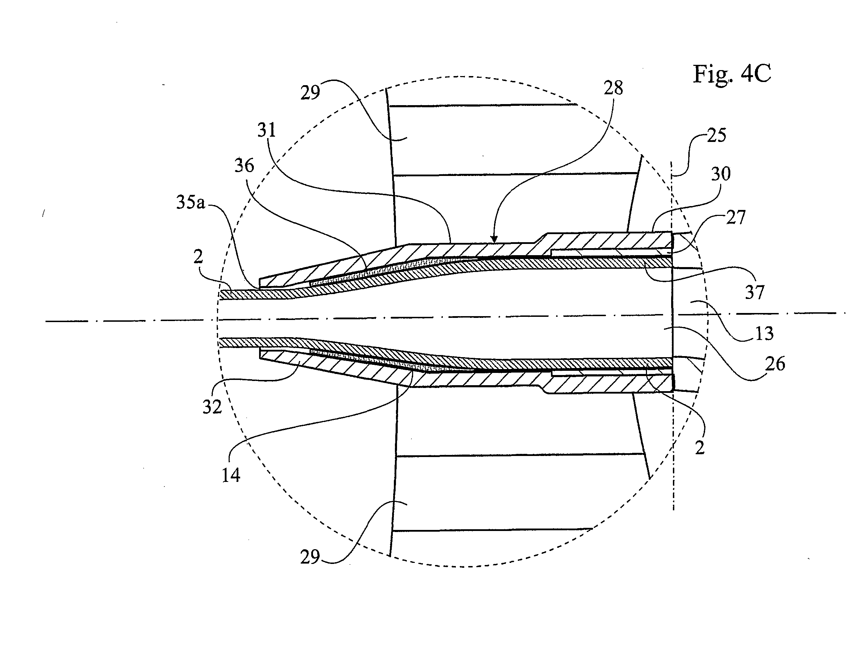

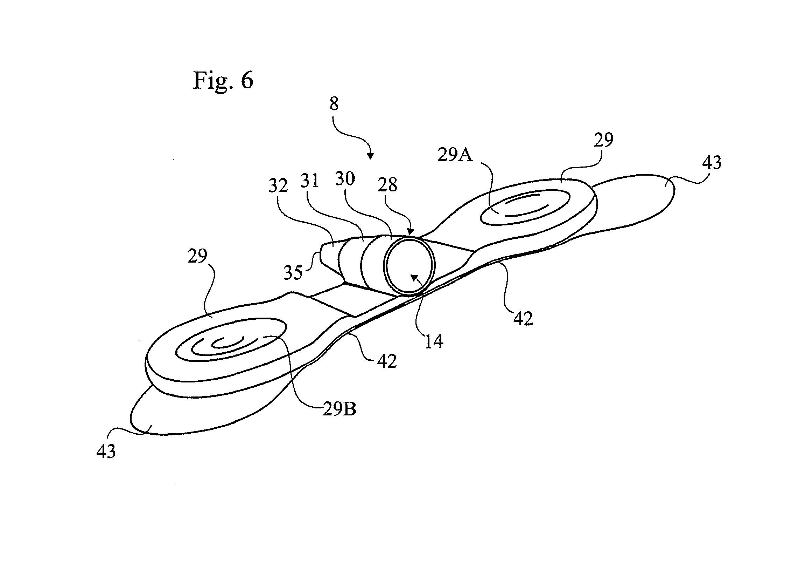

The distal portion 11 of the hub 4 is this text, as far as the present embodiment is concerned, is defined as the portion the rear limit of which is defined by the plane 24, which is defined as the mouth 26 of the branch conduit 13 in front of the curve 15, said plane 25 being perpendicular to the cannula passageway 12 and to the centre line 69 of the distal portion 11. The distal portion 11 according to the embodiment consists of a rear portion in the form of an annular, cylindrical neck 27, which projects forwards from the rear portion 10 a distance in the axial direction of the hub 4, and the unit 8 which is shown in Fig. 6 and which is provided with said gripping and fixation member 7. said unit 8 consisting of a sleeve 28, which is provided with a pair of wings 29, which project in the lateral directions from the bottom side of the sleeve. The sleeve 28 consists of a cylindrical collar 30 in the rear portion of the sleeve 28, a cylindrical central portion 31, and a nose cone 32. The sleeve 28 is connected to the rear portion 10 of the hub by means of the collar 30, which is mounted snug fit on the neck 27 and is fixed to the neck according to any technique which may be conventional, e.g. through press fit, gluing or other adhesive connection, or through HF-welding. According to the embodiment, however, gluing is preferably employed. In Fig. 4C, a glue joint is designated 36. The inner radius of the neck 27 is slightly larger then the radius of the mouth 26 of the branch conduit. The difference approximately corresponds to the wall thickness of the catheter 2. In the region of its central portion 31, and in its nose cone 32, the sleeve 28 is provided with a first, cylindrical space and with a conical tapered space, respectively. In combination, the conical, tapered space, the first cylindrical space in the central portion 31, and a second cylindrical space in the cylindrical interior of the neck 28 form said inlet chamber 14. In the front end, a circular opening 35 is provided for the catheter 2.

If the catheter 2 consists of a polymeric material which does not swell in contact with liquids of the human body, such as blood, the diameter of the circular opening 35 corresponds with the outer diameter of the catheter 2. However, the catheter 2 preferably consists, at least partly, of a hydrophilic plastic material which swells significantly in contact with body liquids, including blood. More particularly, the catheter 2 according to the preferred embodiment is made of a composite plastic material of the type which is mentioned in the introductory description of the invention and which will be described more in detail in the following. In this case, the outer diameter of the catheter is initially smaller than the diameter of the opening 35, so that the catheter is allowed to swell freely in use without being prevented by the material of the hub, more particularly by the material of the sleeve 28 in the region of the opening 35. Fig 4C illustrates the conditions before the catheter 2 has started swelling causing an increase of its diameter in the region of the opening 35. In order to make said increase of the diameter of the catheter 2 possible, there is initially an annular gap 35a between the catheter 2 and the sleeve 28. The width of the gap 35a is sufficiently large to allow the catheter 2 to increase its diameter without obstacle when blood or other dialyse liquid flows through the catheter. Suitably, the width corresponds to the increase of the diameter of the catheter because of the swelling, in other words that the catheter due to the swelling eliminates the gap without pressing itself with any considerable pressure against the sleeve 28 in the region of the opening 35.

The catheter 2 extends through the opening 32 and, according to the embodiment, through the inlet chamber 14 all the way to said plane 25; in other words to the mouth 26 of the branch conduit 13. At least in the region of the nose cone 32 and/or in the first cylindrical space, the diameter of the catheter increases gradually in the longitudinal direction of the catheter from the opening(preferably the increase of diameter begins already in the region of the conical space, such that the terminal portion 37 of the catheter has a width corresponding to the inside of the neck 27 against which said terminal portion 37 is attached. Also for this attachment, any of the techniques which have been mentioned above in connection with the fixation of the sleeve 28 to the neck 27 can be employed, namely press fit, adhesive connection/gluing or HF-welding, which all can be employed for the polymeric material that the catheter and the hub 4 are made of.

As has been described in the foregoing, the catheter 2 according to the embodiment consists of one or more polyurethane based materials, while the hub 4 may consist for example of any of the following materials: PC (polycarbonate), HDPE (high density polyethene), PU (polyurethane), PVC (polyvinyl chloride), COPP (copolymer polypropene). Through the described mounting of the catheter 2 in the proximate portion 11 of the hub 4, there is created an even passageway without sudden changes of the cross section and therefore an even flow path for the blood that shall be conveyed through the catheter 2 and through the branch conduit 13 during the performance of haemodialysis. Not even in the transition between the catheter 2 and the branch conduit 13 in the region of the mouth 26 of the branch conduit there is any significant change of the cross section of the passageway since the inner diameter of the catheter 2 in the region of the neck 27 essentially corresponds to the diameter of the branch conduit 13 in the region of the mouth 26. This contributes to a careful transportation of the blood liquid which reduces the risk of haemolysis because of turbulence in the liquid.

The wings 29 have two functions. Firstly, they shall be used as gripping means when the cannula 3 together with the catheter 2 shall penetrate a fistula 40. Secondly, they shall fix the hub 4 and the catheter 2 to the body of the patient during the dialysis treatment, which normally has a duration of at least 4-5 hours. It is known per se to provide a catheter assembly with wing-like gripping members, which also can be used to fix a catheter and a hub to the patient. In order to improve these functions in accordance to known technique, the wings 29 have been made larger and the very gripping portion has been given a shape which is adapted to be gripped between the thumb and another finger. Further, the wings have been placed in the region of the distal portion 11 of the hub, in other words adjacent to the catheter and to the cannula 3, which promotes accuracy at the penetration of the fistula 40. In order to prevent that the wings 29 will slide relative to one another during the handling of the assembly 1 in connection with the penetration of the fistula, one of the wings is provided with a convex protrusion 29A and the other wing is provided with a correspondingly shaped concavity 29B. When the person who handles the assembly 1 presses the two wings 29 together, the protrusion 29A thus is pressed into the concavity 29B. On the opposite side, i.e. on the side which forms the bottom surface, which shall be applied against the patient's skin, the wings 29 are provided with an adhesive coating, which is protected by a pair of pieces 42 of a plastic foil, which can be released. Among conceivable adhesives may be mentioned for example "double coated spun lace non woven" tape 9917, which is manufactured by 3M Co. Each piece of plastic foil 42 is provided with a pull tab 43, which extends a distance outwards rearwards. The plastic foil pieces 42 cover the entire bottom surfaces 29 and meet edge-edge on the bottom surface of the assembly 1. The contact edges are designated 44. As a further measure in forming a safe grip for the person who shall handle the assembly 1, the outer surface of the covering plastic foil pieces 42 have been given an uneven screen pattern 45.

During transportation and storage, the point of the cannula 3 and the catheter 2 are covered by a protecting cap 47, which is conventional. The cap 47 is releasably pressed against the nose cone 32.

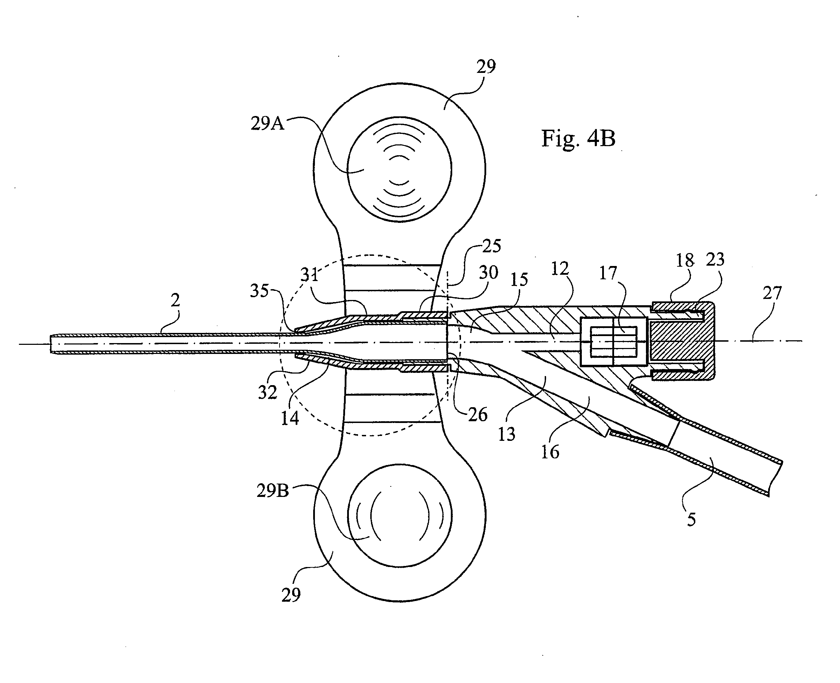

In the rear end of the proximate conduit 5, there is also a rear gripping member 48, here referred to as rear wing. The rear wing 48 consists of a comparatively flat object having a thin front male portion 49, which is pressed into the rear end of the proximate conduit 5, and a rear male portion 50 with an external thread, which forms part of a luer-lok™ coupling including the lid 18, which in a subsequent phase shall be transferred to the rear portion 10 of the hub 4, Fig. 4B, if that is desired. A passageway 51 extends through the rear wing 48. On the proximate conduit 5, there is a clamp 52 of a type known per se. The edges of the rear wing are rounded. Due to the fact that the rear wing 48 is flat but nevertheless is void of any sharp edges, it is adapted to be employed as a gripping member as well as a fixing member for fixing also the rear part of the catheter assembly against the patient's skin. According to the embodiment, a piece of tape is used for fixing the rear wing 48 against the skin, but also adhesive coatings on at least one of the two flat sides of the wing 48, covered by pieces of protecting foils, are conceivable.

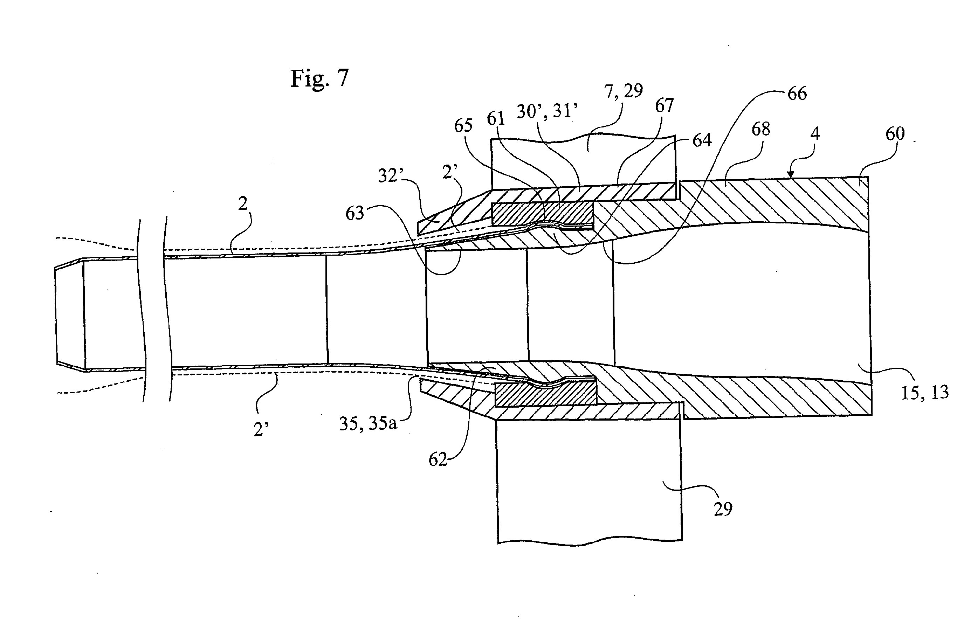

Fig. 7 shows the distal portion of the catheter assembly 1' according to a second embodiment of the invention in an axial cross section at a larger scale. The assembly includes a catheter 2 of a composite plastic material, which is designed such that the catheter will swell in the radial but not in the axial direction in contact with blood or other aqueous liquid according to the principles which have been briefly described in the foregoing and which will be described more in detail in the following. Further, a hub 4' with a main body 60 and a sleeve 28' are included. A front section 66 of the main body 60 extends all the way, or nearly all the way to the opening 35 of the hub. The assembly also includes a slotted metal or plastic ring 61, which is included in a mechanical coupling for fixing the catheter 2 in the hub 4'. In other respects, the hub 1' is designed in the same mode as that embodiment which has been described with reference to Fig. 1-6. Thus the sleeve 28' is provided with a pair of wings 29 which are included in a gripping and fixing member 7.

The front section 66 of the main body 60 is tapered stepwise in the direction towards the opening 35. A terminating portion 62 has a conical outer surface and a cylindrical inner surface, such that it in cross section has the shape of a wedge. The inner surface 63 has a diameter which essentially corresponds with the inner diameter of the swollen catheter 2' (dash lined), when blood or other aqueous liquid has passed through the catheter for a period of time. A projecting bead 64 is provided on the outer side of the conical noose portion 62.

A rear fastening section 2a of the catheter is widened, mechanically or through heating or through a combination of heating and mechanical widening, and mounted on the conical nose portion 62, which forms part of the main body 60 of the hub 4'. The catheter then has been clamped on the conical nose portion 62 by means of the ring 61, which is slotted and thus possible to clamp, and which is provided with an inner, circumferential groove matching the bead 64 on the conical portion 62. Subsequently the sleeve 28' is mounted over the ring 61, which is covered by the front portion 31' of a cylindrical portion, the rear section 30' of which extends over an annular recess 67 on the main body 60 of the hub 4'. The sleeve 28' is terminated by a nose cone 32'. In the same mode as in connection with the foregoing embodiment, a narrow, annular gap 35a is provided between the nose cone 32' and the catheter 2 prior to swelling of the catheter 2.

The swollen state of the catheter 2 is indicated through dash lines. As has been mentioned in the foregoing, the swollen catheter has an inner diameter which essentially corresponds with the inner diameter of the inside 63 of the terminating section of the nose cone 32. Thereafter, the diameter increases successively, without any sudden changes of the cross section of the passageway which extends through the hub through the main body 60 of the hub 4' and which proceeds via the curve 15 into the branch conduit 13.

Through the embodiment which is shown in Fig. 7 and described above, the catheter 2 is secured safely in the hub 4' as wall in the swollen as in the non-swollen state of the catheter. At the same time, also this embodiment provides a very even increase of the diameter of the passageway which extends through the catheter assembly, which reduces the risk of turbulence in the medium which shall flow through the assembly.

When the catheter assembly 1 or 1' shall be used, the protective cap 47 is first removed. The handling person holds the assembly 1 with the wings 29, which are brought against one another, in a finger grip, Fig. 7. The protrusion 29A in engagement with the concavity 29B prevents the wings from moving relative to one another at the same time as the surface structure 45 on the pieces of plastic foils 42 counteracts that the wing will slide between the fingers, even if the operator would use gloves of a smooth material. By means of the gripping member 7, which is established by the wings 29, which are provided adjacent to the catheter and to the cannula 3, the cannula 3 and the catheter 2 therefore can be caused to penetrate the fistula wall and be inserted into the fistula 40 with small risk of undesired movements of the assembly 1, which could cause damage to the fistula.

As soon as the catheter 2 has been introduced into the fistula, the covering plastic foil pieces 42 on the wings 29 can be removed by means of the pull tags 43, and the wings 29, which extend sideways from the bottom surface of the sleeve 28, can be fixed against the patient's skin by means of the adhesive on the wings 29.

When blood has been detected in the chamber 19, the cannula 3 can be removed and the luer-lok™ closure 18 be transferred to the rear end 23 on the hub 4 if that is desired. Thereafter, the operator may ease the clamp 52, so that blood is allowed to flow into the branch conduit 13 and into the proximate conduit 5, whereupon a hose 53 to/from the dialysis apparatus can be connected. At such connection of a vein or artery catheter to the corresponding vein and artery hoses of the dialysis apparatus, the rear wing 48 facilitates the opening of the luer-lok™ connection 18, Fig. 1, as well as the connection of the hose 53 via a luer-lok™ coupling 18' to the proximate conduit 5, which also promotes a careful handling of the entire assembly and hence a reduced risk that the catheter 2 would be disturbed or subjected to stresses which could damage the fistula. As has been mentioned above, the rear wing 48 can be used also for fixing the catheter assembly 1, 1', the proximate conduit 5 and the hose 53 against the skin of the patient by means of a piece of tape or adhesive coating on either side of the rear wing, whose rounded edges do not chafe or in any other way are inconvenient to the patient.

## Claims

1. Catheter tubing (2), **characterised in that** it consists of a composite comprising a first material which consists substantially of a hydrophilic plastic material which absorbs moist and therefore swells such that its volume will increase significantly in contact with e.g. blood, and a second material which essentially does not absorb moist and therefore will not swell in a moist environment, but which forms reinforcing, longitudinal strands (55) in the catheter tubing, which counteract that the catheter tubing is elongated or that its thickness is increased due to the swelling capacity of the first polymeric material, that also the first material forms a longitudinal strands (54) in the tubing, and that the strands of said first and second materials, in a cross section of the tubing, form edge connected segments, such that the increase of the volume of the hydrophilic first plastic material substantially results in an increase of the circumference of the tubing and hence of its diameter and duct cross section area.

2. Catheter tubing according to claim 1, **characterised in that** each segment consisting of a swelling plastic material is arranged between a pair of segments of non-swelling material and vice versa.

3. Catheter tubing according to claim 2, **characterised in that** the number of segments of each plastic material is at least ten, preferably at least fifteen, suitably at least twenty.

4. Catheter tubing according to claim 3, **characterised in that** the width of the swellable segments is not larger than the wall thickness of the catheter tubing, preferably smaller.

5. Catheter tubing according to any of claims 1-4, **characterised in that** the swelling and non-swelling plastic materials belong to the same family of plastic materials, comprising polyurethanes and polyamides.

6. Catheter tubing according to claim 5, **characterised in that** the plastic materials are of a polyurethane type.

7. Catheter tubing according to any of claims 1-6, **characterised in that** the swellable strands (54) in total initially represent 2-98 % of the circumference, preferably 5-50 % of the circumference, suitably 15-40 % of the circumference.

8. Catheter tubing according to any of claims 1-7, **characterised in that** the stiffness of said second material expressed in Pa at 20 °C is at least 10⁸ Pa, preferably at least 5 x 10⁸ Pa, most advantageously at least 10 x 10⁸ Pa, and that the stiffness ratio 20 °C/36 °C, i.e. the ratio between the stiffnesses at 20 °C and 36 °C, is not more than 2, preferably not more than 1.5.

9. Catheter tubing according to any of the previous claims, **characterised in that** any of said swellable and non-swellable strands, which have the shape of segments of an annular ring in cross section, preferably the non-swelling strands, contain reinforcing strands of stronger material embedded in the plastic material of said segments of an annular ring

10. Catheter tubing according to any of the previous claims, **characterised in that** the swellable plastic material has a maximum swelling capacity amounting 800 %, preferably not more than 200 %, suitably not more than 100 %.

11. Catheter tubing according to claim 10, **characterised in that** the swelling capacity is at least 10 %, preferably at least 15 %.

12. Catheter tubing according to claim 11, **characterised in that** the swelling capacity is 15-60 %.

13. Catheter tubing **characterised in that** it is made of a tubing according to any of claims 1-12.

## Patentansprüche

1. Katheterröhre (2), **dadurch gekennzeichnet, dass** sie aus einem Verbundwerkstoff besteht, welcher ein erstes Material, das im Wesentlichen aus einem hydrophilen Kunststoffmaterial besteht, das Feuchtigkeit absorbiert und dadurch quillt, so dass sein Volumen in Kontakt mit z. B. Blut signifikant zunehmen wird, und ein zweites Material umfasst, das im Wesentlichen keine Feuchtigkeit absorbiert und daher in einer feuchten Umgebung nicht quellen wird, jedoch verstärkende, längsgerichtete Stränge (55) in der Katheterröhre bildet, die der Längung der Katheterröhre oder deren Dickenanstieg infolge der Quellkapazität des ersten polymeren Materials entgegenwirken, dass das erste Material auch längsgerichtete Stränge (54) in der Röhre bildet und dass die Stränge der ersten und zweiten Materialien in einem Querschnitt der Röhre an ihren Kanten verbundene Segmente bilden, so dass der Anstieg des Volumens des hydrophilen ersten Kunststoffmaterials im Wesentlichen zu einem Anstieg des Umfangs der Röhre und somit ihres Durchmessers und ihrer Leitungsquerschnittsfläche führt.

2. Katheterröhre nach Anspruch 1, **dadurch gekennzeichnet, dass** jedes Segment, das aus einem quellenden Kunststoffmaterial besteht, zwischen einem Paar von Segmenten aus nicht-quellendem Material angeordnet ist, und anders herum.

3. Katheterröhre nach Anspruch 2, **dadurch gekennzeichnet, dass** die Anzahl der Segmente jedes Kunststoffmaterials mindestens zehn, vorzugsweise mindestens fünfzehn, geeigneterweise mindestens zwanzig beträgt.

4. Katheterröhre nach Anspruch 3, **dadurch gekennzeichnet, dass** die Breite der quellbaren Segmente nicht größer als die Wanddicke der Katheterröhre ist, vorzugsweise kleiner.

5. Katheterröhre nach einem der Ansprüche 1 bis 4, **dadurch gekennzeichnet, dass** die quellenden und nicht-quellenden Kunststoffmaterialien zu derselben Familie von Kunststoffmaterialien gehören, die Polyurethane und Polyamide umfasst.

6. Katheterröhre nach Anspruch 5, **dadurch gekennzeichnet, dass** die Kunststoffmaterialien vom Polyurethantyp sind.

7. Katheterröhre nach einem der Ansprüche 1 bis 6, **dadurch gekennzeichnet, dass** die quellbaren Stränge (54) insgesamt anfangs 2 bis 98 % des Umfangs, vorzugsweise 5 bis 50 % des Umfangs, geeigneterweise 15 bis 40 % des Umfangs ausmachen.

8. Katheterröhre nach einem der Ansprüche 1 bis 7, **dadurch gekennzeichnet, dass** die in Pa angegebene Steifheit des zweiten Materials bei 20 °C mindestens 10⁸ Pa, vorzugsweise mindestens 5 x 10⁸ Pa, besonders vorteilhaft mindestens 10 x 10⁸ Pa beträgt, und dass das Steifheitsverhältnis von 20 °C/36 °C, d. h. das Verhältnis zwischen der Steifheit bei 20 °C und der Steifheit bei 36 °C, nicht mehr als 2, vorzugsweise nicht mehr als 1,5 beträgt.

9. Katheterröhre nach einem der vorhergehenden Ansprüche, **dadurch gekennzeichnet, dass** jedwede der quellbaren und nicht-quellbaren Stränge, die die Form von Segmenten eines annularen Rings im Querschnitt haben, vorzugsweise die nicht-quellenden Stränge, verstärkende Stränge aus stärkerem Material enthalten, die in das Kunststoffmaterial der Segmente eines annularen Rings eingebettet sind.

10. Katheterröhre nach einem der vorhergehenden Ansprüche, **dadurch gekennzeichnet, dass** das quellbare Kunststoffmaterial eine maximale Quellkapazität hat, die sich auf 800 % beläuft, vorzugsweise nicht mehr als 200 %, geeigneterweise nicht mehr als 100 %.

11. Katheterröhre nach Anspruch 10, **dadurch gekennzeichnet, dass** die Quellkapazität mindestens 10 %, vorzugsweise mindestens 15 % beträgt.

12. Katheterröhre nach Anspruch 11, **dadurch gekennzeichnet, dass** die Quellkapazität 15 bis 60 % beträgt.

13. Katheterröhre, **dadurch gekennzeichnet, dass** sie aus einer Röhre nach einem der Ansprüche 1 bis 12 hergestellt ist.

## Revendications

1. Tubage de cathéter (2), **caractérisé en ce qu'**il est constitué d'un composite comprenant un premier matériau qui est constitué essentiellement d'une matière plastique hydrophile qui absorbe l'humidité et donc gonfle de telle sorte que son volume augmente de façon significative en contact avec, par exemple, du sang, et un second matériau qui n'absorbe pratiquement pas l'humidité et qui ne gonfle pas dans un environnement humide, mais qui forme des brins longitudinaux de renforcement (55) dans le tubage de cathéter, qui font obstacle à ce que le tube de cathéter soit allongé ou à ce que son épaisseur soit augmentée en raison de la capacité de gonflement du premier matériau polymère, **en ce que** le premier matériau forme également des brins longitudinaux (54) dans le tubage, et **en ce que** les brins des premier et second matériaux, en section transversale du tuyau, forment des segments reliés par les bords, de telle sorte que l'augmentation du volume du premier matériau plastique hydrophile se traduit essentiellement en une augmentation de la circonférence du tubage et par conséquent de son diamètre et de la surface du conduit en section transversale.

2. Tubage de cathéter selon la revendication 1, **caractérisé en ce que** chaque segment se composant d'un matériau de plastique gonflant est disposé entre une paire de segments de matériau non gonflant, et vice versa.

3. Tubage de cathéter selon la revendication 2, **caractérisé en ce que** le nombre de segments de chaque matière plastique est d'au moins dix, de préférence d'au moins quinze, et de manière appropriée, d'au moins vingt.

4. Tubage de cathéter selon la revendication 3, **caractérisé en ce que** la largeur des segments pouvant gonfler n'est pas supérieure à l'épaisseur de la paroi du tubage de cathéter, et de préférence est inférieure.

5. Tubage de cathéter selon l'une quelconque des revendications 1 à 4, **caractérisé en ce que** les matériaux plastiques gonflants et non gonflants appartiennent à la même famille de matériaux plastiques, comprenant les polyuréthanes et les polyamides.

6. Tubage de cathéter selon la revendication 5, **caractérisé en ce que** les matériaux plastiques sont du type polyuréthane.

7. Tubage de cathéter selon l'une quelconque des revendications 1 à 6, **caractérisé en ce que** les brins gonflables (54) représentent au départ un total de 2 à 98 % de la circonférence, de préférence de 5 à 50 % de la circonférence, et de façon appropriée, de 15 à 40 % de la circonférence.

8. Tubage de cathéter selon l'une quelconque des revendications 1 à 7, **caractérisé en ce que** la rigidité dudit deuxième matériau exprimée en Pa à 20°C est d'au moins 10⁸ Pa, de préférence d'au moins 5 x 10⁸ Pa, et le plus avantageusement d'au moins 10 x 10⁸ Pa, et **en ce que** le rapport de la rigidité à 20°C/36°C, c'est à dire le rapport entre les raideurs à 20°C et 36°C, n'est pas supérieur à 2, et de préférence pas supérieur à 1,5.

9. Tubage de cathéter selon l'une quelconque des revendications précédentes, **caractérisé en ce que** chacun desdits brins gonflables et non gonflables, qui ont la forme de segments d'un anneau circulaire en section transversale, et de préférence les brins non gonflables, contient des brins de renfort en un matériau plus résistant noyé dans le matériau plastique desdits segments d'un anneau circulaire.

10. Tubage de cathéter selon l'une quelconque des revendications précédentes, **caractérisé en ce que** le matériau plastique susceptible de gonfler a une capacité de gonflement s'élevant au maximum à 800 %, de préférence pas supérieure à 200 %, et de manière appropriée pas supérieure à 100 %.

11. Tubage de cathéter selon la revendication 10, **caractérisé en ce que** la capacité de gonflement est d'au moins 10 %, de préférence d'au moins 15 %.

12. Tubage de cathéter selon la revendication 11, **caractérisé en ce que** la capacité de gonflement est comprise entre 15 et 60 %.

13. Tubage de cathéter **caractérisé en ce qu'**il est constitué d'un tubage selon l'une quelconque des revendications 1 à 12.
